# EUROPÄISCHE PATENTANMELDUNG

(11) **EP 3 400 914 A1**
(43) Veröffentlichungstag der Anmeldung: **14.11.2018**
(21) Anmeldenummer: 17169996.0
(22) Anmeldetag: 08.05.2017
(51) Int. Cl.: A61F 2/962, A61F 2/95

(54) **HANDGRIFF FÜR EINEN KATHETER SOWIE ENTSPRECHENDER KATHETER**

(71) Anmelder: Biotronik AG, 8180 Bülach (CH)
(72) Erfinder: Westhoff, Felix, 8057 Zürich (CH); Rothfuchs, Nuria, 8032 Zürich (CH)
(74) Vertreter: Galander, Marcus

(57) **Zusammenfassung**

Die Erfindung betrifft einen Handgriff für einen Katheter (1) mit einem Innenschaft (9) und einem zurückziehbaren Schaft (8), wobei der Handgriff (2)
- ein Gehäuse (11),
- ein Zugseil (18) und
- eine an dem Gehäuse (11) beweglich gelagerte Sicherungstaste (4) aufweist.

Um ein Umwickeln des Zugseils um den Innenschaft zu vermeiden ist in dem Gehäuse (11) eine Hülse (16) befestigt, in deren durchgehender Öffnung (21) ein Bremsenschlauch (17) in Längsrichtung beweglich geführt ist, wobei der Bremsenschlauch (17) eine erste durchgehende Öffnung (31) zur Anordnung des Zugseils (18) und eine zweite durchgehende Öffnung (32) zur Anordnung des Innenschafts (9) aufweist, wobei der Bremsenschlauch (17) derart eingerichtet ist, dass seine Position relativ zu der Hülse (16) in einem ersten Zustand mittels der Sicherungstaste (4) festgelegt ist und dass der Bremsenschlauch (17) in einem zweiten Zustand nach Betätigen der Sicherungstaste (4) zusammen mit dem Zugseil (18) in Längsrichtung der Hülse (16) und relativ zu der Hülse (16) und dem Innenschaft (9) in proximaler Richtung zum Zurückziehen des zurückziehbaren Schafts (8) verschiebbar ist, wobei der zurückziehbare Schaft (8) mit dem Zugseil (18) verbindbar ist.

Die Erfindung betrifft ferner einen Katheter (1) mit einem solchen Handgriff (2) sowie ein Verfahren zum Betreiben eines derartigen Katheters (1).

## Beschreibung

Die Erfindung betrifft einen Handgriff für einen Katheter mit einem Innenschaft und einem zurückziehbaren Schaft, wobei der Handgriff ein Gehäuse und eine an dem Gehäuse beweglich gelagerte Sicherungstaste aufweist.

Als Katheter werden im Allgemeinen Röhrchen oder Schläuche unterschiedlichen Durchmessers aus verschiedenen Materialien bezeichnet, mit denen Hohlorgane wie Harnblase, Magen, Darm, Blutgefäße oder das Herz sondiert, entleert, gefüllt, gespült oder anderweitig minimalinvasiv behandelt werden können. Der in den zu behandelnden Körper eines Menschen oder Tiers einzuführende rohr- oder schlauchförmige Abschnitt des Katheters wird als Schaft bezeichnet, an dessen distalem Ende die Katheterspitze angeordnet ist, welche von den Katheterelementen am weitesten in den zu behandelnden Körper eindringt. Entsprechend werden Richtungsangaben wie distal bzw. proximal im Rahmen dieser Anmeldung immer im Sinne von näher an der Katheterspitze bzw. näher am Behandler verstanden.

Es sind bereist intravaskuläre Katheter, vor allem für die Anwendung im Herz-Thoraxbereich, mit einer aktiven oder passiven Elektrode bekannt, welche in Hauptvenen oder -arterien, beispielsweise in die Vena Femoralis, eingeführt und von dort aus an unterschiedliche Stellen im Herzen bzw. zu den Herzkranzgefäßen vorgeschoben werden können. Diese Katheter werden dafür verwendet, die elektrische Aktivität des Herzens darzustellen oder zu stimulieren oder Bereiche mit abnormaler elektrischer Aktivität abzutragen. Die zuletzt genannte Ablationstherapie dient z.B. als Therapie gegen Herzrhythmusstörungen. Ferner werden heutzutage Katheter z. B. dafür eingesetzt, um mittels Neuromodulation der renalen Nerven (plexus renalis) eine Blutdrucksenkung zu erreichen. Weitere Beispiele für Katheter sind solche, die zum Einsetzen eines Stents dienen und/oder an ihrer Katheterspitze einen Ballon aufweisen, der, wenn er dilatiert wird, zum Aufweiten eines Gefäßes oder Expandieren eines Stents an der zu behandelnden Stelle eingesetzt werden kann. Zum Freisetzen einer Einrichtung wie eines Stents besitzt ein Katheter häufig einen Innenschaft und einen den Innenschaft umgebenden zurückziehbaren Schaft.

Der Schaft eines derartigen Katheters ist in der Regel an seinem proximalen Ende mit einem Handgriff zur Kathetersteuerung und ggf. der Fluidversorgungssteuerung verbunden. Beispielsweise wird mit dem Handgriff das Zurückziehen des zurückziehbaren Schafts mittels eines Zugseils bewirkt. Handgriffe bekannter Katheter besitzen häufig das Problem, dass sich beim Zurückziehen des Zugseils dieses um den Innenschaft des Katheters wickelt. Hierdurch wird die Funktion des Katheters beeinträchtigt.

Aus der Druckschrift DE 10 2005 051 469 A1 ist eine Vorrichtung zum Einführen eines selbstexpandierenden Stents in ein Körpergefäß bekannt, welche an ihrem proximalen Ende einen Griff mit einer Durchführung aufweist, über welche ein Schiebeelement und ein Drahtführungskatheter am Griff befestigt sind, und mit einem Hebel, welcher mit dem Griff über ein Drehgelenk verbunden ist. Das Schiebeelement dient zur Stabilisierung des Stents beim Zurückziehen eines Schlauchs von dem Drahtführungskatheter. Weiter ist ein Arretierknopf vorgesehen, der eine Relativbewegung zwischen einem Schlauch und einem Schiebelement verhindert. Nach Entriegeln des Arretierknopfs kann durch Betätigung des Hebels mit jedem Betätigungshub ein Hüllschlauch um die Strecke eines Zahnabstandes einer Zahnstange, die ebenfalls am Griff befestigt ist, zurückgezogen werden.

Aus der Druckschrift DE 198 15 119 C1 ist eine Antriebsvorrichtung für eine elastische Sonde, insbesondere medizinische Sonde, bekannt. Die drahtförmige Sonde wird durch eine vordere Öffnung in die Antriebsvorrichtung eingelegt und liegt auf einem Steg auf. Der Steg weist eine gekrümmte Auflagefläche zur besseren seitlichen Fixierung der Drahtsonde auf. Die Drahtsonde wird anschließend durch einen Griff der Antriebsvorrichtung an der proximalen Seite wieder herausgeführt. Ferner ist an der Antriebsvorrichtung ein Rad mit einer zirkulären umfangsseitigen konischen, sich zur Mitte hin verjüngenden Nut vorgesehen, wobei die Nutweite und die Position von Schlitzen an der Antriebsvorrichtung, in welche seitliche Achsbolzen des Rads eingepasst sind, so gewählt sind, dass die Sonde von den Nutwänden des Rads gegen den Steg gedrückt und so in der Nut reibschlüssig gehalten wird.

Die obigen Druckschriften liefern keine Lösung zu dem oben angegebenen Problem.

Die Aufgabe besteht somit darin, einen Handgriff für einen Katheter zu schaffen, der ein Umwickeln des Zugseils um den Innenschaft vermeidet sowie die Handhabbarkeit des Katheters verbessert. Die Aufgabe besteht ferner darin, einen Katheter mit einem solchen Handgriff zu schaffen und ein einfaches Verfahren zum Betreiben eines solchen Katheters anzugeben, das das obige Problem vermeidet.

Die obige Aufgabe wird durch den Handgriff mit den Merkmalen des Anspruchs 1 sowie den Katheter mit den Merkmalen des Anspruchs 11 gelöst.

Insbesondere ist in dem Gehäuse des Handgriffs erfindungsgemäß eine hohlzylindrische Hülse befestigt, in deren durchgehender Öffnung (Lumen) ein Bremsenschlauch in Längsrichtung beweglich geführt ist, wobei der Bremsenschlauch eine erste durchgehende Öffnung (Lumen) zur Anordnung des Zugseils des Handgriffs und eine zweite durchgehende Öffnung (Lumen) zur Anordnung des Innenschafts des Katheters aufweist. Erfindungsgemäß ist der Bremsenschlauch derart eingerichtet, dass seine Position relativ zu der Hülse (und dem Gehäuse) des Handgriffs in einem ersten Zustand mittels der Sicherungstaste festgelegt ist und dass der Bremsenschlauch in einem zweiten Zustand nach Betätigen der Sicherungstaste zusammen mit dem Zugseil in Längsrichtung der Hülse und relativ zu der Hülse und dem Innenschaft in proximaler Richtung zum Zurückziehen des zurückziehbaren Schafts verschiebbar ist, wobei der zurückziehbare Schaft mit dem Zugseil verbindbar ist. Im ersten Zustand, welcher der Ausgangszustand der Sicherungstaste darstellt, können sich demnach der Bremsenschlauch sowie das in diesem festgelegte Zugseil nicht bewegen. Der oben angegebene zweite Zustand des Bremsenschlauchs tritt ein, wenn die Sicherungstaste betätigt wurde und der Bremsenschlauch hierdurch freigegeben wurde.

Eine durchgehende Öffnung in einem Schlauch, einem Schaft oder einer Hülse wird gewöhnlich auch als (Innen)Lumen des Schlauches, des Schaftes oder der Hülse bezeichnet. Dieses Lumen bzw. die durchgehende Öffnung ist ein innerer Hohlraum, der von dem Schlauch, dem Schaft oder der Hülse umschlossen wird.

Die erste und die zweite durchgehende Öffnung des Bremsenschlauchs liegen in dem Bremsenschlauch bezogen auf den Querschnitt nebeneinander. Hieraus ergibt sich, dass der Bremsenschlauch in einem Ausführungsbeispiel einen von einer Kreisform abweichenden Querschnitt aufweist. Hierdurch wird eine Rotation des Bremsenschlauchs in der Hülse erschwert.

Der erfindungsgemäße Handgriff hat weiter den Vorteil, dass durch die Anordnung des Zugseils und des Innenschaftes in getrennten Öffnungen (Lumen) des Bremsenschlauchs ein Umwickeln des Zugseils um den Innenschaft verhindert wird. Zudem wird der Innenschaft während des Freisetzens z. B. eines Stents am distalen Ende des Katheters (Katheterspitze) durch den Bremsenschlauch und die Hülse geführt. Dies stabilisiert den Innenschaft. Vorzugsweise ist das Zugseil als Zugdraht ausgebildet. Das Zugseil ist fest in der ersten Öffnung des Bremsenschlauchs angeordnet und so mit dem Bremsenschlauch verbunden.

Die Hülse besitzt vorzugsweise eine Länge (Abmessung in Längsrichtung der Hülse), welche größer ist als die Länge des freizusetzenden Implantats. Dies bedeutet, dass die Hülse über das distale Ende des Handgriffs hinausstehen kann. Das Implantat kann beispielsweise ein selbstexpandierender Stent sein. Bei Systemen geeignet für bis zu 200mm lange Stents kann die Gesamtlänge (Handgriff (ca 150mm) + Hülse außerhalb des Handgriffs (ca. 100mm)) der Hülse zum Beispiel 250 mm betragen. Hierdurch wird eine bessere Führung des Zugseils und der Katheterschäfte sowie eine Stabilisierung des Teils des Katheters erreicht, welcher im Gebrauchszustand außerhalb des Patienten bleibt.

In einem Ausführungsbeispiel weist der Handgriff eine am Gehäuse befestigte und drehbar gelagerte Rolle auf, an welcher das Zugseil befestigt ist und auf welche das Zugseil (bzw. das Zugseil mit dem Bremsenschlauch) beim Verschieben in proximaler Richtung aufwickelbar ist. Mit der Rolle, die durch einen entsprechende Öffnung in dem Gehäuse des Handgriffs von außen zugänglich und durch den Benutzer drehbar ist, lässt sich das Zugseil mit dem Bremsenschlauch einfach aus der Hülse herausziehen. Da das Zugseil an dem zurückziehbaren Schaft des Katheters befestigt ist, ist hierdurch der zurückziehbare Schaft einfach relativ zu dem Innenschaft verschiebbar. Vorzugsweise ist die Rolle an dem distalen Ende des Handgriffs angeordnet, um es einfach bedienen zu können. Das in Richtung des proximalen Endes des Handgriffs durchlaufende Zugseil sowie der Bremsenschlauch werden vorzugsweise am proximalen Ende des Handgriffs mittels eines am Gehäuse angebrachten Umlenkstegs oder einer am Gehäuse drehbar gelagerten und am Gehäuse befestigten Umlenkrolle mit wenig Reibung in Richtung der Rolle zum Aufwickeln umgelenkt, so dass die Führung von Bremsenschlauch und Zugseil im Gehäuse des Handgriffs weiter verbessert ist.

In einem weiteren bevorzugten Ausführungsbeispiel weist die Hülse eine im Querschnitt ovale durchgehende Öffnung zur Anordnung des Bremsenschlauchs auf, wobei sich der große Durchmesser des ovalen Querschnitts in Richtung der nebeneinander liegenden ersten und zweiten Öffnungen des Bremsenschlauchs erstreckt. Die ovale Öffnung der Hülse erschwert eine Rotation des im Querschnitt nicht rotationssymmetrischen Bremsenschlauchs in der Hülse zusätzlich. Ferner wird eine Drehmomentübertragung vom Handgriff auf das Schlauchsystem des Katheters ermöglicht.

Es ist weiter von Vorteil, wenn die Festlegung der Position des Bremsenschlauchs in dem ersten Zustand mittels eines Form- und/oder Kraftschlusses zwischen der Sicherungstaste und dem Bremsenschlauch erfolgt. Hierfür weist die Sicherungstaste beispielsweise einen U-förmigen Abschnitt, vorzugsweise mit mindestens einem an einer Seitenfläche des U-förmigen Abschnitts angeordneten zahnförmigen Vorsprung und/oder mindestens eine Schneide, auf. Der U-förmige Abschnitt besitzt vorzugsweise einen Innendurchmesser, der kleiner ist als der Durchmesser des Bremsenschlauchs in die gleiche Richtung, so dass auf den Bremsenschlauch, wenn dieser in dem Schlitz angeordnet ist, mittels Kraft- und/oder Formschluss eine Kraft einwirkt, welche den Bremsenschlauch in dem Gehäuse bzw. in der Hülse festlegt.

Einen besonders effektiven Kraft- und/oder Formschluss kann die Sicherungstaste im ersten Zustand erzielen, wenn die Hülse in dem Bereich, in dem der Formschluss zwischen der Sicherungstaste und dem Bremsenschlauch in dem ersten Zustand herstellbar ist, eine Ausnehmung derart aufweist, dass der Bremsenschlauch im Bereich der Ausnehmung freiliegt. Alternativ kann die Hülse in diesem Bereich unterbrochen sein. Die Sicherungstaste wirkt somit unmittelbar auf den Bremsenschlauch.

In einem weiteren Ausführungsbeispiel wird das Trennen (Peelen) des Bremsenschlauchs von dem Innenschaft des Katheters verbessert, wenn in dem Mantel des Bremsenschlauchs ein bis zur zweiten Öffnung durchgehender Schlitz vorgesehen ist. Der Schlitz verläuft somit radial zu dem Bremsenschlauch bzw. in Bezug auf den Innenschaft. Vorzugsweise erstreckt sich der Schlitz entlang der gesamten Länge des Bremsenschlauchs.

In einer Weiterbildung der Erfindung enthält das Material des Bremsenschlauchs Teflon und/oder ein anderes Fluorpolymer und/oder eine thermoplastische Verbindung. Insbesondere Teflon weist gute Gleiteigenschaften auf, so dass das Zurückziehen des Bremsschlauchs in der Hülse sowie um den Umlenksteg oder die Umlenkrolle mit wenig Kraftaufwand und somit präzise durchgeführt werden kann.

In einem weiteren bevorzugten Ausführungsbeispiel ist vorgesehen, dass die Sicherungstaste ein vorzugsweise fingerförmiges Sperrelement aufweist, welches in dem ersten Zustand eine Drehung der Rolle, auf die das Zugseil beim Zurückziehen aufgewickelt wird, sperrt und in dem zweiten Zustand eine Drehung der Rolle erlaubt. Das Sperrelement ist im ersten Zustand beispielsweise mittels eines Formschlusses mit der Rolle verbunden, während im zweiten Zustand dieser Formschluss nicht mehr besteht. Z. B. greift eine Spitze, die an dem am weitesten von der Sicherungstaste entfernten Ende des Sperrelements ausgebildet ist, in ein gezahntes Rad ein, das an der Rolle neben der Gehäusewand vorgesehen ist. Hierdurch wird die Bewegung des gezahnten Rads und, da es starr mit der Rolle verbunden ist, der Rolle verhindert. Im zweiten Zustand wird der Eingriff des Sperrelements in das gezahnte Rad durch Betätigung der Sicherungstaste aufgehoben und das gezahnte Rad sowie die Rolle können sich frei drehen. Hierdurch wird der Handgriff auf einem zusätzlichen Weg vor dem versehentlichen Zurückziehen des Zugseils und somit des rückziehbaren Schafts gesichert.

In einem weiteren Ausführungsbeispiel des erfindungsgemäßen Handgriffs weist die Sicherungstaste ein Rastelement auf, welches beim Betätigen der Sicherungstaste in ein entsprechendes Rast-Gegenelement, das am Gehäuse angeordnet oder befestigt ist, einrastet. Das Rastelement bildet einen Formschluss mit einem Rast-Gegenelement. Das Rastelement ist beispielsweise als ein an der Sicherungstaste befestigter fingerförmiger Vorsprung mit einem Haken an dem vorderen Ende ausgebildet. Das Rast-Gegenelement ist ein an dem Gehäuse angebrachter, vorzugsweise mit dem Gehäuse einstückiger, Steg, der durch den Haken im zweiten Zustand umgriffen wird. Die Sicherungstaste wird durch das Rastelement und das Rast-Gegenelement in dem zweiten Zustand gehalten.

Es ist für die Montage des Katheters mit Handgriff vorteilhaft, wenn die Sicherungstaste die Hülse weitgehend umgreift, wobei an der Sicherungstaste eine sich entlang der Längsrichtung der Hülse erstreckende Öffnung vorgesehen ist, welche zum Einlegen der Hülse mit Zugseil und Innenschaft dient. Indem die Hülse in die Sicherungstaste anstelle von Einführen eingelegt werden kann, wird die Montagezeit beträchtlich verkürzt.

Die obige Aufgabe wird auch durch einen Katheter mit einem zurückziehbaren Schaft und einem Innenschaft sowie einen oben beschriebenen Handgriff gelöst, wobei der Innenschaft in dem zurückziehbaren Schaft angeordnet ist. Zusätzlich kann an dem Katheter ein Außenschaft vorgesehen sein. Der erfindungsgemäße Katheter besitzt die oben zu dem Handgriff beschriebenen Vorteile.

Die oben genannten Vorteile ergeben sich insbesondere für einen Katheter, wo ein (bevorzugt selbstexpandierendes) Implantat auf dem Innenschaft angeordnet ist und durch die Relativbewegung des zurückziehbaren Schaftes freigesetzt wird. Derartige Implantate können Herzklappenprothesen mit einem selbstexpandierenden Grundgerüst, elektrodenlose Schrittmacher (leadless pacer), selbstexpandierende Verschlußsysteme und insbesondere selbstexpandierende Stents sein.

In einem Ausführungsbeispiel ist das Zugseil an dem zurückziehbaren Schaft und der Innenschaft an dem Gehäuse des Handgriffs befestigt. Hierdurch kann der zurückziehbare Schaft durch das Zugseil in proximale Richtung verschoben werden. Auch eine Verschiebung in distale Richtung ist möglich, wenn das Zugseil entsprechend steif ausgebildet ist. Die Befestigung des Zugseils an dem zurückziehbaren Schaft erfolgt vorzugsweise mittels einer entsprechend Hülse, wobei im Ausgangszustand (d. h. vor Betätigung der Sicherungstaste) der Ort der Befestigung in der Hülse des Handgriffs, und zwar vorzugsweise an dem distalen Ende der Hülse, angeordnet ist.

Falls der Katheter einen Außenschaft aufweist, so ist dieser vorzugsweise an der Hülse des Handgriffs, und zwar an deren distalem Ende befestigt, beispielsweise stoffschlüssig mit der Hülse verbunden, z. B. mittels Schweißen.

Der zusätzliche Außenschaft, häufig auch als Stabilisatorschaft bezeichnet, umschließt den zurückziehbaren Schaft und den darin angeordneten Innenschaft auf den größten Teil ihrer Länge. Er dient der Verbesserung der Einführbarkeit in den Patienten und der Stabilisierung des Gesamtsystems, insbesondere währende der Freisetzung des Implantats.

Ein einfaches und kostengünstiges sowie zuverlässiges Verfahren zum Betreiben eines oben beschriebenen Katheters, beispielsweise zum Freisetzen eines selbstexpandierenden Stents beinhaltet die Schritte, dass die Sicherungstaste betätigt wird und dass anschließend der Bremsenschlauch zusammen mit dem darin angeordneten Zugseil in eine Längsrichtung der Hülse und relativ zu der Hülse und dem Innenschaft in proximaler Richtung verschoben wird. Wenn der Handgriff eine mit dem Zugseil verbundene Rolle aufweist, erfolgt das Verschieben des Bremsenschlauchs relativ zur Hülse des Handgriffs in proximaler Richtung mittels Drehen der an dem Gehäuse des Handgriffs drehbar gelagerten Rolle zum Aufwickeln des Zugseils und/oder des Bremsenschlauchs. Das Zurückziehen des Zugseils wird demnach mittels Drehen an der Rolle durchgeführt. Die Rolle weist hierfür beispielsweise an einem äußeren Rand eines mit der Rolle starr verbundenen Drehrads Riffelungen auf, die ein Drehen der Rolle erleichtern. Alternativ oder zusätzlich wird die Rolle aus einem weichen Material (beispielsweise ein weicher Kunststoff) ausgeführt, welches ebenfalls für eine höhere Reibung zwischen Rolle und Zugseil sorgt.

Weitere Ziele, Merkmale, Vorteile und Anwendungsmöglichkeiten der Erfindung ergeben sich auch aus der nachfolgenden Beschreibung von Ausführungsbeispielen der Erfindung anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch:
- Fig. 1: einen Katheter mit einem Handgriff in einer Ansicht von der Seite,
- Fig. 2: den Schaft des Katheters gemäß Fig. 1 in einem Querschnitt,
- Fig. 3: einen erfindungsgemäßen Handgriff bei geöffnetem Gehäuse,
- Fig. 4: eine vergrößerte Darstellung des geöffneten Gehäuses des Handgriffs gemäß Fig. 3,
- Fig. 5: die Sicherungstaste mit Bremsenschlauch des Handgriffs gemäß Fig. 3 in einer perspektivischen Ansicht von der Seite,
- Fig. 6: die Anordnung gemäß Fig. 5 in einer Ansicht von der Seite und
- Fig. 7: die Hülse mit Bremsenschlauch, Innenschaft und Zugseil des Handgriffs gemäß Fig. 3 in einem Querschnitt.

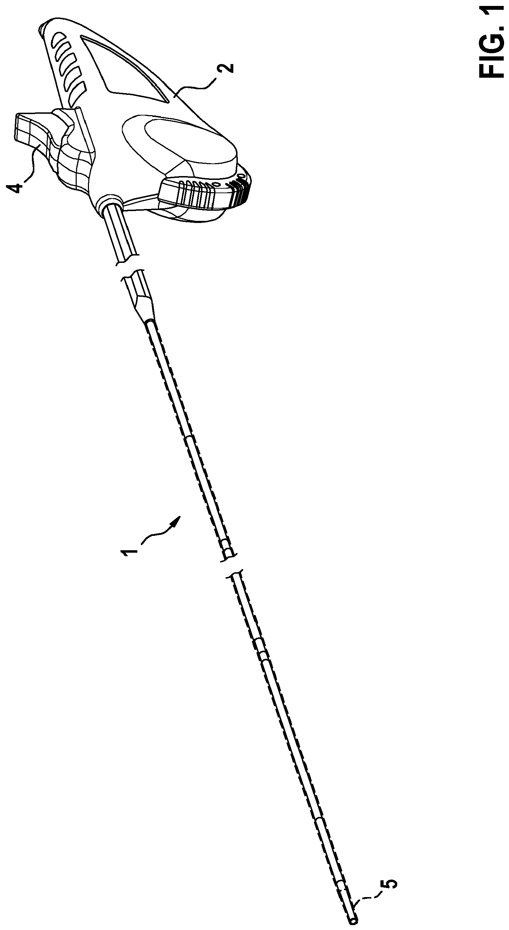

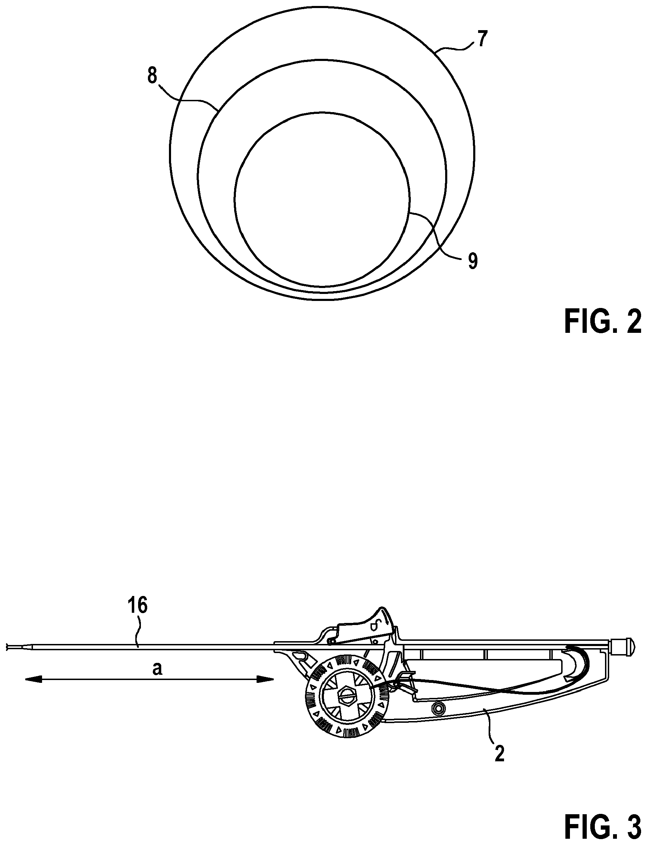

Fig. 1 zeigt ein Beispiel eines Katheters 1 mit einem proximal angeordneten Handgriff 2. Der Handgriff 2 besitzt eine Sicherungstaste 4. Über den einhändig bedienbaren Handgriff 2 lässt sich ein an der Katheterspitze 5, d.h. an dem äußersten distalen Ende des Katheters, angeordnetes Objekt, beispielsweise ein selbstexpandierender Stent, positionieren und freisetzen. Die Freisetzung erfolgt beispielsweise mittels eines triaxialen Schaftsystems, das in Fig. 2 dargestellt ist. Die Freisetzung erfolgt unter anderem durch Betätigen der Sicherungstaste 4, welche eine versehentliche Freisetzung verhindert.

Der triaxiale Schaft des Katheters 1, bestehend aus einem Außenschaft 7, einem zurückziehbaren Schaft 8 und einem Innenschaft 9, ist in Fig. 2 im Querschnitt dargestellt. Andere Schaftbauweisen sind ebenfalls denkbar. Der Innenschaft 9 ist innerhalb des zurückziehbaren Schafts 8 angeordnet, welcher sich innerhalb des Außenschaftes 7 befindet. Um den Innenschaft 9 und damit das nicht dargestellte einzubringende Objekt freizusetzen, wird der zurückziehbare Schaft 8 nach proximal verschoben.

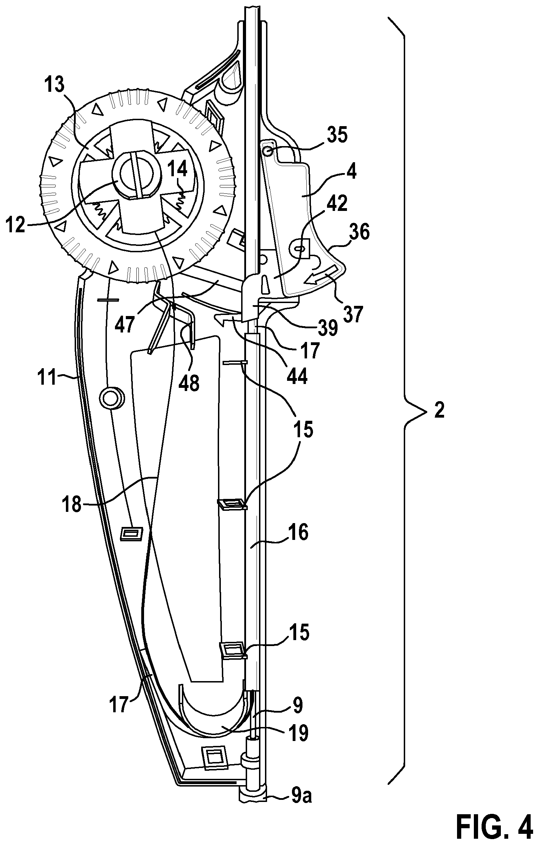

In den Fig. 3 bis 7 ist der erfindungsgemäße Handgriff 2 im Detail gezeigt. Dieser Handgriff aus Fig. 1 und 2 stellt eine Ausgestaltung des erfindungsgemäßen Kathetersystems dar. Das Gehäuse 11 besteht aus zwei lösbar zusammenfügbaren Hälften, wobei in Fig. 3 und 4 nur eine Gehäusehälfte sichtbar ist, wobei die Gehäusehälften beispielsweise mittels einer Schraubverbindung fest miteinander verbunden werden können. Alternativ können die beiden Hälften auch über eine Klemmverbindung miteinander verbunden sein.

Am proximalen Ende des Gehäuses 11 ist eine Rolle 12 zum Aufwickeln eines Zugseils 18 (beispielsweise als Zugdraht verwirklicht) vorgesehen. Die Rolle 12 ist an dem Gehäuse drehbar gelagert befestigt und starr mit einem Rad 13 verbunden, mit dem ein Benutzer die Rolle 12 drehen kann. Hierfür weist das Rad 13 an seinem äußeren Umfang eine entsprechende Riffelung sowie Pfeile auf, welche die richtige Drehrichtung zum Aufwickeln des Zugseils 18 kennzeichnen. Das rollenseitige Ende des Zugseils 18 ist fest mit der Rolle 12 verbunden. Mit der Rolle 12 ist ferner ein gezahntes Rad 14 fest verbunden, welches zwischen dem Rad 13 zum Drehen durch den Benutzer und dem Gehäuse 11 angeordnet ist. Das gezahnte Rad 14 ist derart ausgestaltet, dass es nur in eine Richtung drehbar ist.

Auf die Rolle 12 wird nach wenigen Umdrehungen des Rades 13 nicht zur das Zugseil 18 sondern die Kombination aus Zugseil 18 und Bremsenschlauch 17 aufgewickelt. Dies geht mit einer entsprechenden Zunahme des aufzunehmenden Materials für die Rolle 12 einher. Vorteilhafterweise wird hier die Rolle 12 konisch ausgestaltet. Für die konkrete Ausgestaltung der Rolle kommen mehrere Möglichkeiten in Frage, die in EP 2 111 826 beschrieben sind, deren Inhalt hiermit einbezogen wird.

An der Innenseite des Gehäuses 11 sind zudem querliegende Verstrebungen 15 vorgesehen, welche eine Hülse 16 am Gehäuse 11 festlegen. Die Hülse 16 ragt, wie in Fig. 3 gezeigt ist, mit einer Länge a über das proximale Ende des Gehäuses 11 des Handgriffs 2 hinaus. Hierdurch wird eine zusätzliche Führung des Katheterschafts beim Zurückziehen des Zugseils 18 erreicht.

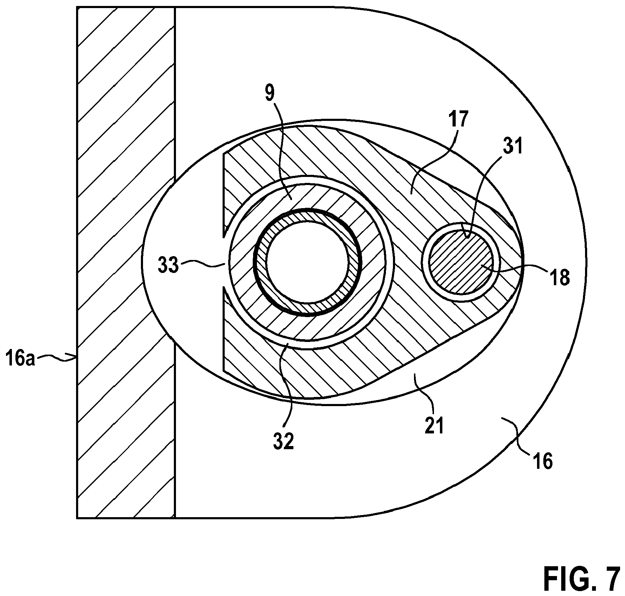

Die Hülse 16 ist in Fig. 7 im Querschnitt zu sehen. Die Hülse 16 besitzt eine ovale durchgehende Öffnung (Lumen) 21 für die Aufnahme eines Bremsenschlauchs 17. Die Hülse 16 ist im Querschnitt auf einer Außenseite abgerundet, während auf der gegenüberliegenden Seite eine gerade Anlagefläche 16a zur Anlage an dem Gehäuse 11 vorgesehen ist.

Der Bremsenschlauch 17 besitzt eine kleinere durchgehende erste Öffnung 31 für die Anordnung des Zugseils 18 und eine mit einem Schlitz 33 versehene, größere zweite Öffnung 32 für die Aufnahme des in der zweiten Öffnung 32 befindlichen Innenschafts 9. Aufgrund der Anordnung der Öffnungen 31, 32 nebeneinander in dem Bremsenschlauch 17 ergibt sich eine nicht rotationssymmetrische Form für den Bremsenschlauch 17.

Die geschlitzte Öffnung 32 des Bremsenschlauchs 17 ermöglicht ein einfaches Aufbringen auf und Lösen vom Innenschaft 9. Der Innenschaft 9 wird entlang der gesamten Hülse 16 vom Bremsenschlauch 17 geführt, so dass er unter Druck nicht ausweichen kann. Lediglich am proximalen Ende des Innenschafts 9 ragt der Innenschaft 9 aus der Hülse 16 hinaus. Am proximalen Ende weist der Innenschaft 9 einen Fluidanschluß 9a zur Spülung der durchgehenden Öffnung des Innenschafts (Innenlumen). Die durchgehende Öffnung des Innenschafts dient der Aufnahme eines Führungsdrahtes, welcher ebenfalls proximal aus dem Innenschaft geführt wird (nicht im Detail dargestellt). In diesem Bereich wird beim Zurückziehen des Zugseils 18 mit dem Bremsenschlauch 17 der Bremsenschlauch 17 von dem Innenschaft 9 automatisch abgezogen (gepeelt), was aufgrund des Schlitzes 33 einfach erfolgen kann. Das automatische Abziehen des Bremsenschlauchs 17 von dem Innenschaft erfolgt durch das Herumführen von Bremsenschlauch 17 um einen Umlenksteg 19, der am proximalen Ende in dem Gehäuse 11 vorgesehen ist.

Die nicht rotationssymmetrische Form des Bremsenschlauchs 17 führt in Kombination mit der ovalen Öffnung 21 der Hülse 16 dazu, dass der Bremsenschlauch 17 nicht in der Hülse 16 rotieren kann. Hierdurch und aufgrund der getrennten Führung von Zugdraht 18 und Innenschaft 9 in getrennten Öffnungen 31, 32 wird ein Umschlingen von Zugdraht 18 und Innenschaft 9 verhindert.

Innerhalb des Gehäuses 11 wird der Bremsenschlauch 17 überwiegend von der Hülse 16 in der ovalen Öffnung 21 (siehe Fig. 7) geführt. Um die Reibung zu minimieren, besteht der Bremsenschlauch 17 aus einem reibungsarmen Material, beispielsweise Teflon. Die Hülse 16 weist im Bereich eines U-förmigen Abschnitts 39 der Sicherungstaste 4 eine Ausnehmung auf oder ist dort, wie in Fig. 4 zu erkennen, unterbrochen. Dadurch liegt der Bremsenschlauch 17 in diesem Bereich frei, so dass ein direktes Zusammenwirken des Bremsenschlauchs 17 mit der Sicherungstaste 4 ermöglicht wird.

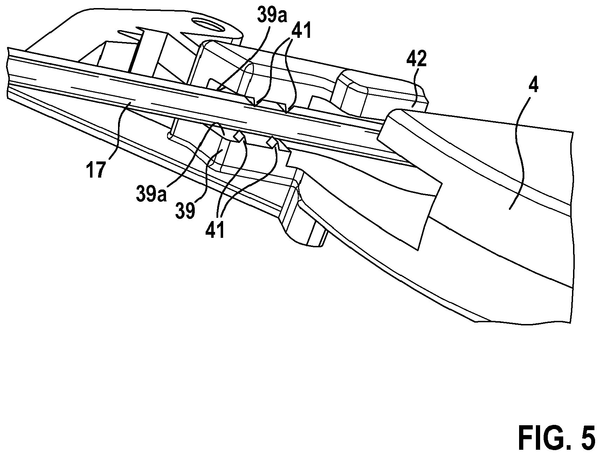

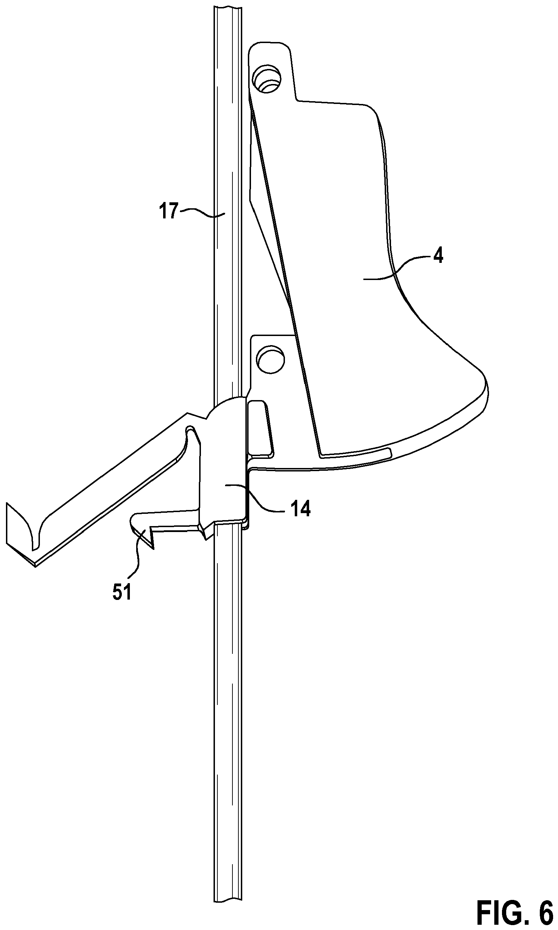

In Fig. 5 und 6 ist die Sicherungstaste 4 detaillierter lediglich mit dem Bremsenschlauch 17 dargestellt. Die Hülse 16 wurde weggelassen. Die Sicherungstaste 4 hat im Wesentlichen eine umgedrehte L-Form und ist schwenkbar um einen vom Gehäuse 11 nach Innen vorstehenden Stift 35 an dem Gehäuse 11 befestigt. Auf der Oberseite, am langen Schenkel des "L", weist die Sicherungstaste 4 eine Mulde 36 auf, in welche der Nutzer ein Finger zum Betätigen der Sicherungstaste 4 durch Herunterdrücken (in Richtung des Pfeils 37 auf der Sicherungstaste 4) legen kann. Im Bereich der Hülse 16 bzw. des Bremsenschlauchs 17 bildet die Sicherungstaste 4 einen U-förmigen Abschnitt aus, welcher die Hülse 16 bzw. den Bremsenschlauch 17 umgreift. An inneren Seitenflächen 39a des U-förmigen Abschnitts 39 sind jeweils zwei Schneiden 41 ausgebildet, welche in einem ersten Zustand mit dem Bremsenschlauch 17 einen Form- und Kraftschluss ausbilden und den Bremsenschlauch 17 relativ zum Gehäuse 11 festlegen, so dass sich der Bremsenschlauch nicht verschieben kann.

An der Sicherungstaste 4 ist ferner eine seitliche Ausnehmung 42 zum Einlegen des Bremsenschlauchs 17 bzw. der Hülse 16 vorgesehen. Die Sicherungstaste 4 weist ferner unterhalb des U-förmigen Abschnitts 39 ein Rastelement in Form eines Hakens 44 sowie ein fingerförmiges Sperrelement 47 auf.

In einem ersten Zustand (Ausgangszustand, Sicherungstaste 4 in der oberen Position) bilden die Schneiden 41 einen Kraft- und Formschluss zwischen der Sicherungstaste 4 und dem Bremsenschlauch 17. Dies erzeugt die Bremswirkung und legt den Bremsenschlauch relativ zum Gehäuse 11 und der Hülse 16 fest. Entsprechend ist das Zugseil 18, das fest in dem Bremsenschlauch 17 angeordnet ist, ebenfalls festgelegt und ein Zurückziehen des Zugseils 18 ist nicht möglich. In dem ersten Zustand greift ferner das fingerförmige Sperrelement 47 mit seinem vorderen Ende in das gezahnte Rad 14 ein, so dass das gezahnte Rad 14 und somit Rolle 12 und Rad 13 nicht gedreht werden können.

Wird die Sicherungstaste 4 durch Drücken in Richtung des Pfeils 37 in die untere Position gebracht, so ist die "Bremse" gelöst und der Kraft- und Formschluss zwischen den Schneiden 41 und dem Bremsenschlauch 17 aufgehoben. Die Sicherungstaste 4 befindet sich nun in dem zweiten Zustand, in dem der Bremsenschlauch 17 in der Hülse 16 verschiebbar ist. In diesem Zustand kann das Objekt an der Katheterspitze 5, beispielsweise ein Stent, freigesetzt werden, indem durch Drehen des Rads 13 das Zugseil 18 zurückgezogen wird. Am Ende der Betätigungsbewegung der Sicherungstaste 14 greift der Haken 44 hinter ein Rast-Gegenelement (Steg 48 am Gehäuse 11). Hierdurch wird die Sicherungstaste 4 in dem zweiten Zustand festgehalten und ein freies Verschieben des Bremsenschlauchs 17 in der Hülse 16 ist ständig gewährleistet. Ferner wird beim Betätigen der Sicherungstaste 4 das Sperrelement 47 beispielsweis durch Knicken an einer Gelenkstelle so bewegt, dass das Sperrelement 47 sich nicht mehr im Eingriff mit dem gezahnten Rad 14 befindet und somit dieses freigibt. Hierdurch können die mit dem gezahnten Rad 14 fest verbundenen Elemente Rolle 12 und Rad 13 gedreht werden, so dass das mit der Rolle 12 verbundene Zugseil 18 zurückgezogen werden kann.

Um ein axiales Verschieben von Zugseil 16 und Bremsenschlauch 17 zu verhindern, kann optional direkt proximal der Stelle des Form- und Kraftschlusses zwischen Bremsenschlauch und "Bremse" ein zylindrischer Überwurf auf den Bremsenschlauch 17 gepresst werden. In dieser optionalen Ausgestaltung der Erfindung erhöht sich so die Haftreibung zwischen Bremsenschlauch 17 und Zugseil 18.

Da das Zugseil 18 im distalen Bereich der Hülse 16 mit dem zurückziehbaren Schaft 8 des Katheters verbunden ist, wird durch Drehen an dem Rad 13 über das Zugseil 18 ebenfalls der zurückziehbare Schaft 8 zurückgezogen. Der Innenschaft 9 bleibt fest in dem Gehäuse 11 und der Außenschaft 7 ist im distalen Bereich der Hülse 16 befestigt. Da die Hülse 16 bezogen auf das Gehäuse 11 ebenfalls festliegt, wird beim Zurückziehen des Zugseils 18 auch der Außenschaft 7 nicht bewegt. Alle drei Schäfte werden zumindest in den distalen Bereich der Hülse 16 hinein geführt, jedoch lediglich der Innenschaft 9 passiert die Hülse 16 über ihre gesamte Länge.

Der erfindungsgemäße Katheter 1 mit dem erfindungsgemäßen Handgriff 2 erlaubt ein sicheres Zurückziehen des zurückziehbaren Schafts 8 mittels des Zugseils 18 und vermeidet ein Umwickeln des Zugseils 18 um den Innenschaft 9. Der Handgriff 2 erlaubt zudem eine Drehmomentübertragung vom Handgriff 2 auf die Schäfte 7 bis 9.

**Bezugszeichenliste:**

| | | | |
|---|---|---|---|
| 1 | Katheter | 41 | Schneide |
| 2 | Handgriff | 42 | Ausnehmung |
| 4 | Sicherungstaste | 44 | Haken |
| 5 | Katheterspitze | 47 | Sperrelement |
| 7 | Außenschaft | 48 | Steg |
| 8 | zurückziehbarer Schaft | | |
| 9 | Innenschaft | | |
| 9a | Stopfen | | |
| 11 | Gehäuse | | |
| 12 | Rolle | | |
| 13 | Rad | | |
| 14 | gezahntes Rad | | |
| 15 | Verstrebung | | |
| 16 | Hülse | | |
| 16a | Anlagefläche der Hülse 16 | | |
| 17 | Bremsenschlauch | | |
| 18 | Zugseil | | |
| 19 | Umlenksteg | | |
| 21 | durchgehende Öffnung (Lumen) der Hülse 16 | | |
| 31 | erste Öffnung für den Zugdraht 18 | | |
| 32 | zweite Öffnung für den Innenschaft 9 | | |
| 33 | Schlitz | | |
| 35 | Stift | | |
| 36 | Mulde | | |
| 37 | Pfeil | | |
| 39 | U-förmiger Abschnitt | | |
| 39a | innere Seitenfläche der U-Form | | |
| a | Länge der Hülse 16 außerhalb des Gehäuses 11 des Handgriffs 2 | | |

## Patentansprüche

1. Handgriff für einen Katheter (1) mit einem Innenschaft (9) und einem zurückziehbaren Schaft (8), wobei der Handgriff (2)
- ein Gehäuse (11),
- ein Zugseil (18) und
- eine an dem Gehäuse (11) beweglich gelagerte Sicherungstaste (4) aufweist,
**dadurch gekennzeichnet, dass** in dem Gehäuse (11)
- eine Hülse (16) befestigt ist, in deren durchgehender Öffnung (21)
- ein Bremsenschlauch (17) in Längsrichtung beweglich geführt ist, wobei der Bremsenschlauch (17) eine erste durchgehende Öffnung (31) zur Anordnung des Zugseils (18) und eine zweite durchgehende Öffnung (32) zur Anordnung des Innenschafts (9) aufweist, wobei der Bremsenschlauch (17) derart eingerichtet ist, dass seine Position relativ zu der Hülse (16) in einem ersten Zustand mittels der Sicherungstaste (4) festgelegt ist und dass der Bremsenschlauch (17) in einem zweiten Zustand nach Betätigen der Sicherungstaste (4) zusammen mit dem Zugseil (18) in Längsrichtung der Hülse (16) und relativ zu der Hülse (16) und dem Innenschaft (9) in proximaler Richtung zum Zurückziehen des zurückziehbaren Schafts (8) verschiebbar ist, wobei der zurückziehbare Schaft (8) mit dem Zugseil (18) verbindbar ist.

2. Handgriff nach Anspruch 1, **dadurch gekennzeichnet, dass** der Handgriff (2) eine am Gehäuse (11) drehbar gelagerte Rolle (12) aufweist, an welcher das Zugseil (18) befestigt ist und auf welche das Zugseil (18) mit dem Bremsenschlauch (17) beim Verschieben in proximaler Richtung aufwickelbar ist.

3. Handgriff nach einem der vorhergehenden Ansprüche, **dadurch gekennzeichnet, dass** die Hülse (16) eine im Querschnitt ovale durchgehende Öffnung (21) zur Anordnung des Bremsenschlauchs (17) aufweist.

4. Handgriff nach einem der vorhergehenden Ansprüche, **dadurch gekennzeichnet, dass** die Festlegung der Position des Bremsenschlauchs (17) in dem ersten Zustand mittels eines Form- und/oder Kraftschlusses zwischen der Sicherungstaste (4) und dem Bremsenschlauch (17) erfolgt.

5. Handgriff nach Anspruch 4, **dadurch gekennzeichnet, dass** die Hülse (16) in dem Abschnitt (39), in dem der Formschluss zwischen der Sicherungstaste (4) und dem Bremsenschlauch (17) in dem ersten Zustand herstellbar ist, eine Ausnehmung derart aufweist, dass der Bremsenschlauch (17) im Bereich der Ausnehmung freiliegt.

6. Handgriff nach einem der vorhergehenden Ansprüche, **dadurch gekennzeichnet, dass** der Bremsenschlauch (17) in seinem Mantel einen bis zur zweiten Öffnung (32) durchgehenden Schlitz (33) aufweist.

7. Handgriff nach einem der vorhergehenden Ansprüche, **dadurch gekennzeichnet, dass** das Material des Bremsenschlauchs (17) Teflon enthält.

8. Handgriff nach einem der vorhergehenden Ansprüche, **dadurch gekennzeichnet, dass** die Sicherungstaste (4) ein vorzugsweise fingerförmiges Sperrelement (47) aufweist, welches in dem ersten Zustand eine Drehung der Rolle (12) sperrt und in dem zweiten Zustand eine Drehung der Rolle (12) erlaubt.

9. Handgriff nach einem der vorhergehenden Ansprüche, **dadurch gekennzeichnet, dass** die Sicherungstaste (4) ein Rastelement (44) aufweist, welches beim Betätigen der Sicherungstaste (4) in ein entsprechendes Rast-Gegenelement (48), das am Gehäuse (11) angeordnet oder befestigt ist, einrastet.

10. Handgriff nach einem der vorhergehenden Ansprüche, **dadurch gekennzeichnet, dass** die Sicherungstaste (4) die Hülse (16) weitgehend umgreift, wobei an der Sicherungstaste (4) eine sich entlang der Längsrichtung der Hülse (16) erstreckende Öffnung (42) vorgesehen ist, welche zum Einlegen der Hülse (16) mit Zugseil (18) und Innenschaft (9) dient.

11. Katheter mit einem zurückziehbaren Schaft (8) und einem Innenschaft (9) sowie einen Handgriff (2) nach einem der vorhergehenden Ansprüche, wobei der Innenschaft (9) in dem zurückziehbaren Schaft (8) angeordnet ist.

12. Katheter nach Anspruch 11, **dadurch gekennzeichnet, dass** das Zugseil (18) an dem zurückziehbaren Schaft (8) befestigt und der Innenschaft (9) an dem Gehäuse (11) des Handgriffs (2) befestigt ist.

13. Verfahren zum Betreiben eines Katheters nach einem der Ansprüche 11 und 12, **dadurch gekennzeichnet, dass** die Sicherungstaste (4) betätigt wird und anschließend der Bremsenschlauch (17) zusammen mit dem darin angeordneten Zugseil (18) in eine Längsrichtung der Hülse (16) und relativ zu der Hülse (16) und dem Innenschaft (9) in proximaler Richtung verschiebbar ist.

14. Verfahren nach Anspruch 13, **dadurch gekennzeichnet, dass** zum Verschieben des Bremsenschlauchs (17) relativ zur Hülse (16) in proximaler Richtung die an dem Gehäuse (11) des Handgriffs (2) drehbar gelagerte Rolle (12) zum Aufwickeln des Zugseils (18) und/oder des Bremsenschlauchs (17) gedreht wird.
